# EUROPÄISCHE PATENTSCHRIFT

(11) **EP 0 570 774 B1**
(45) Veröffentlichungstag und Bekanntmachung des Hinweises auf die Patenterteilung: **03.01.1996**
(21) Anmeldenummer: 93107398.5
(22) Anmeldetag: 07.05.1993
(51) Int. Cl.: C07C 29/56, C07C 33/035

(54) **Verfahren zur Herstellung von Vinylglykolen, durch Isomerisierung**
Method of preparing vinylglycols by isomerisation
Procédé de préparation de vinylglycols par isomérisation

(30) Priorität: 16.05.1992 DE 4216315
(43) Veröffentlichungstag der Anmeldung: 24.11.1993
(73) Patentinhaber: BASF Aktiengesellschaft, D-67063 Ludwigshafen (DE)
(72) Erfinder: Becker, Rainer, Dr., W-6702 Bad Duerkheim (DE); Gramlich, Walter, Dr., W-6900 Heidelberg (DE); Huellmann, Michael, Dr., W-6148 Heppenheim (DE); Scholz, Hans-Ulrich, Dr., W-6719 Weisenheim (DE)

(56) Entgegenhaltungen:
- EP-A- 0 142 657
- FR-A- 1 155 290

## Beschreibung

Die vorliegende Erfindung betrifft ein verbessertes Verfahren zur Herstellung von Vinylglykolen der allgemeinen Formel (I) 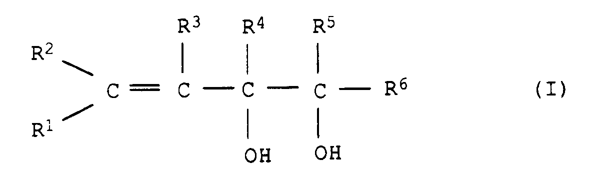
in der die Reste R¹ bis R⁶ unabhängig voneinander gleich oder verschieden oder untereinander verknüpft sein können und H, C₁ bis C₁₀-Alkyl, C₅ bis C₇-Cycloalkyl C₇ bis C₁₀-Aralkyl oder einen ein- oder zweikernigen Arylrest bedeuten, wobei diese Reste auch inerte Substituenten tragen können, durch Isomerisierung der entsprechenden Diole der allgemeinen Formel II 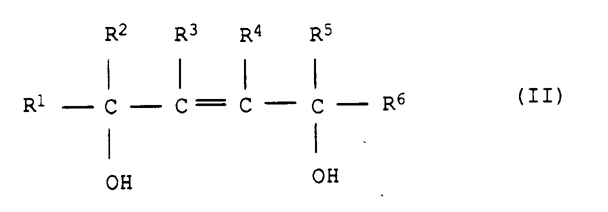
in Gegenwart katalytisch wirkender Substanzen.

Es ist bekannt, daß But-2-en-1,4-diol mittels Metallkatalysatoren in Gegenwart von Säuren in Vinylglykol überführt werden kann (EP-A-142 657, Rao et al, Tetrahedron 45 (22), 7031 (1989)). Da Vinylglycole wie das But-1-en-3,4-diol als sekundäre allylständige Alkohole jedoch sehr säureempfindlich sind, entstehen oft unerwünschte Nebenprodukte. Insbesondere bei der Isolierung der Produkte ergeben sich Probleme, so daß vor der Aufarbeitung der Reaktion die Säure zuerst neutralisiert werden muß. Nach den bekannten Verfahren erhält man das But-1-en-3,4-diol überdies nur in Ausbeuten bis zu etwa 60 %.

Aufgabe der vorliegenden Erfindung war es deshalb, ein wirtschaftliches Verfahren zur Herstellung von Vinylglykolen (I) aus ungesättigten Diolen (II) zu entwickeln.

Demgemäß wurde eine Verbesserung des eingangs definierten Verfahrens gefunden, welche dadurch gekennzeichnet ist, daß man als Katalysator Rhenium oder eine Rheniumverbindung verwendet.

Nach dem erfindungsgemäßen Verfahren lassen sich ungesättigte Diole (II) der allgemeinen Formel (II) isomerisieren, dabei sind die Reste R¹ bis R⁶ unabhängig voneinander gleich oder verschieden. Bevorzugt werden vor allem Wasserstoff und daneben C₁ bis C₁₀-Alkylgruppen, die sowohl linear als auch verzweigt sein können. Unter den Alkylgruppen werden die Methyl- und Ethylgruppe, Propyl-und Butylgruppen bevorzugt. Weiterhin kommen C₅ bis C₇-Cycloalkylgruppen, wie insbesondere eine Cyclopentyl- oder eine Cyclohexylgruppe, ein- oder zweikernige Arylreste, wie Naphtyl und insbesondere Phenyl, sowie C₇- bis C₁₀-Aralkylgruppen, beispielsweise Benzyl, in Betracht. Die genannten Reste können ihrerseits Substituenten tragen, die sich unter den Reaktionsbedingungen inert verhalten, also beispielsweise eine niedere Alkylgruppe oder ein Halogen wie Chlor. Darüber hinaus können cyclische ungesättigte Diole, in denen beispielsweise die Reste R³ und R⁴ miteinander verknüpft sind, eingesetzt werden. Vorzugsweise hat der Ring 5 bis 7 Kohlenstoffatome. Auch dieser Ring kann seinerseits wie oben beschrieben substituiert sein.

Die Diole II sind bekannt oder nach bekannten Methoden erhältlich. So kann man beispielsweise Acetylen mit Aldehyden oder Ketonen umsetzen und die hierbei erhältlichen Derivate des But-2-in-1,4-diols katalytisch partiell hydrieren (s. z.B. Houben Weyl, Bd V/1b, Seite 779, 783, 790, Herausg. Eugen Müller, Georg Thieme Verlag, Stuttgart, 1972).

Erfindungsgemäß erfolgt die Isomerisierung der ungesättigten Diole zu den Vinylglykolen (I) in Gegenwart von Rhenium oder eines Rheniumkatalysators. Als Rheniumkatalysator kann metallisches Rhenium verwendet werden. Vorzugsweise liegt es fein verteilt vor, wobei es auch in Verbindung mit einem Träger eingesetzt werden kann. Ferner kommen als erfindungsgemäße Katalysatoren anorganische sowie organische Rheniumverbindungen in Betracht, beispielsweise Ammoniumperrhenat. Es können auch Mischungen verschiedener Rheniumverbindungen verwendet werden. Besonders bevorzugt wird Rheniumheptoxid eingesetzt. Vorzugsweise wird die Isomerisierung unter neutralen Bedingungen vorgenommen, d.h. dem Reaktionsgemisch wird keine Säure zugesetzt.

Bezogen auf die Menge des eingesetzten Diols (II), beträgt die eingesetzte Katalysatormenge in der Regel nicht weniger als 10 ppm Re, als Metall gerechnet. Die Reaktion führt im allgemeinen bei Katalysatormengen von 0,1 Gew.-% glatt zu den gewünschten Produkten. Es kann aber auch erforderlich sein, den Katalysator in Mengen bis zu etwa 1 Gew.-% einzusetzen. Mengen an katalytisch wirkender Substanz von mehr als 2 Gew.-% bringen im allgemeinen keine Verbesserung mehr.

Die Isomerisierung kann lösungsmittelfrei oder in Gegenwart eines Lösungsmittels durchgeführt werden. Zu den geeigneten Lösungsmitteln zählen C₅- bis C₂₀-Kohlenwasserstoffe wie Hexan, Cyclohexan, Toluol, Xylol oder höhersiedende Paraffine. Es können aber auch halogenierte C₁- bis C₆-Kohlenwasserstoffe wie Methylenchlorid, Chloroform oder Chlorbenzol verwendet werden. Die erfindungsgemäße Isomerisierung kann auch in C₂- bis C₁₂-Alkyl- oder Arylethern, wie Diethylether, Tetrahydrofuran oder Diphenylether ausgeführt werden. Es ist ebenso möglich ein Lösungsmittelgemisch zu verwenden. Es kann aber auch vorteilhaft sein, die Reaktion in der Gasphase an einem Rheniumkatalysator durchzuführen. Vorzugsweise wird die Reaktion in flüssiger Phase vorgenommen.

Im allgemeinen kann die Reaktion bereits bei etwa 20°C durchgeführt werden. Ab etwa 80°C verläuft die Reaktion mit merklicher Geschwindigkeit. Jedoch kann es zweckmäßig sein, Reaktionstemperaturen bis etwa 160°C zu wählen. Höhere Reaktionstemperaturen als etwa 180°C sind meist nicht sinnvoll.

Die Isomerisierungsreaktion kann sowohl diskontinuierlich als auch kontinuierlich durchgeführt werden. Bevorzugt wird die Fahrweise, bei der das Ausgangsdiol (II) dem Reaktionsgemisch kontinuierlich zudosiert und das Vinylglycolprodukt (I) dem Reaktionsgemisch kontinuierlich entzogen wird. In den Fällen, in denen der Siedepunkt der Ausgangsverbindung (II) höher ist als der des Produktes (I) hat es sich als besonders vorteilhaft erwiesen, den Rheniumkatalysator im Lösungsmittel vorzulegen, den Druck auf etwa 0,01 bis etwa 500 mbar zu vermindern und als Reaktionstemperatur die entsprechende Siedetemperatur des Vinylglycolproduktes (1) zu wählen. Während das ungesättigte Diol dem Reaktionsgemisch kontinuierlich zugesetzt wird, kann kontinuierlich fraktioniert abdestilliert werden. Im allgemeinen enthält das so gewonnene Destillat Fraktionen, die reich an Vinylglykolprodukt (I) sind und solche, die dimerisiertes Produkt enthalten. Daher ist es meist zweckmäßig, eine weitere fraktionierte Destillation anzuschließen, um das Dimere thermisch in das gewünschte Produkt zu überführen.

Die Vinylglykole fallen in hoher Reinheit und in hohen Ausbeuten an und dienen als Zwischenprodukte für Wirkstoffe wie Vitamin A oder als Monomere für die Herstellung von hydrophilen Polymerisaten, insbesondere Copolymerisaten.

### Beispiel

Eine Mischung aus 100 g Paraffinöl und 1 g Re₂O₇ wurde auf 130°C erhitzt. Nachdem ein Druck von etwa 0,5 mbar eingestellt worden war, wurden 150 g Buten-2-diol-1,4 innerhalb von ca. 2 Stunden zugegeben, wobei die Reaktionstemperatur bei 130°C gehalten wurde. Gleichzeitig wurde aus der Reaktionsmischung abdestilliert, wobei mehrere Fraktionen mit unterschiedlicher Produktanreicherung erhalten wurden. Eine weitere fraktionierte Destillation ergab 125,1 g Destillat mit einem Gehalt an Buten-1-diol-3,4 von 90,8 %, was einer Ausbeute von 76 % der Theorie entspricht.

## Patentansprüche

1. Verfahren zur Herstellung von Vinylglykolen der allgemeinen Formel (I) 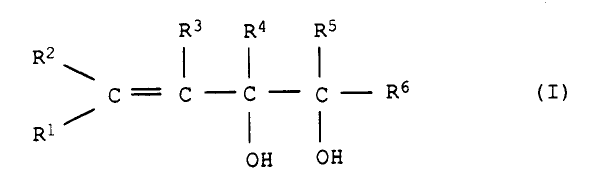 in der die Reste R¹ bis R⁶ unabhängig voneinander gleich oder verschieden oder untereinander verknüpft sein können und H, C₁ bis C₁₀-Alkyl, C₅ bis C₇-Cycloalkyl, C₇- bis C₁₀-Aralkyl oder einen ein- oder zweikernigen Arylrest bedeuten, wobei diese Reste auch inerte Substituenten tragen können durch Isomerisierung der entsprechenden Diole der allgemeinen Formel (II) 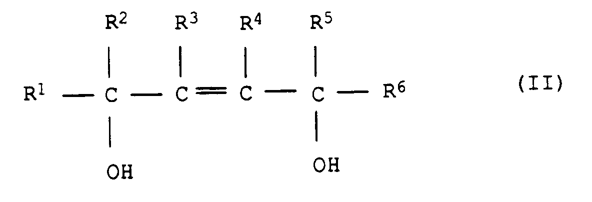 in Gegenwart katalytisch wirkender Substanzen, dadurch gekennzeichnet, daß man als Katalysator Rhenium oder eine Rheniumverbindung verwendet.

2. Verfahren nach Anspruch 1, dadurch gekennzeichnet, daß man die Isomerisierung unter neutralen Bedingungen vornimmt.

3. Verfahren nach Anspruch 1 oder 2, dadurch gekennzeichnet, daß man als ungesättigtes Diol (II) ein ungesättigtes Diol der allgemeinen Formel (II) verwendet, in der die Reste R¹, R², R⁵ und R⁶ Wasserstoff bedeuten.

4. Verfahren nach Anspruch 3, dadurch gekennzeichnet, daß man als ungesättigtes Diol (II) But-2-en-1,4-diol verwendet.

5. Verfahren nach den Ansprüchen 1 bis 4, dadurch gekennzeichnet, daß man als Rheniumkatalysator Rheniumheptoxid verwendet.

6. Verfahren nach den Ansprüchen 1 bis 5, dadurch gekennzeichnet, daß man die Isomerisierung in mindestens einem Lösungsmittel ausgewählt aus der Gruppe der C₅- bis C₂₀-Kohlenwasserstoffe, der halogenierten C₁- bis C₆-Kohlenwasserstoffe und der C₂- bis C₁₂-Alkyl- oder Arylether, durchführt.

7. Verfahren nach den Ansprüchen 1 bis 6, dadurch gekennzeichnet, daß man dem Reaktionsgemisch das entstandene Vinylglykol (I) kontinuierlich destillativ entzieht.

## Claims

1. A process for preparing vinyl glycols of the general formula (I) 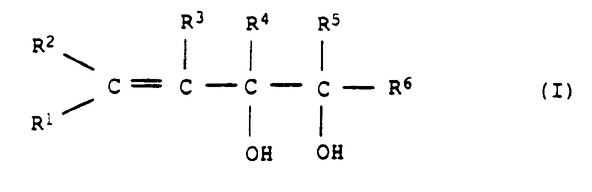 where the radicals R¹ to R⁶ independently of one another can be identical or different or linked to one another and are H, C₁- to C₁₀-alkyl, C₅- to C₇-cycloalkyl, C₇- to C₁₀-aralkyl or a mono- or binuclear aryl radical, it also being possible for these radicals to carry inert substituents, by isomerization of the corresponding diols of the general formula (II) 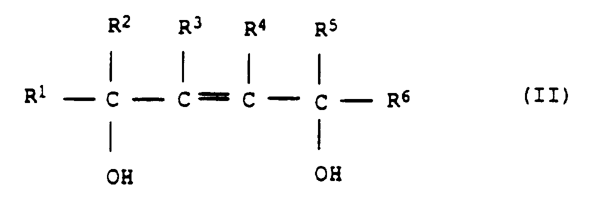 in the presence of substances having a catalytic effect, which comprises using rhenium or a rhenium compound as a catalyst.

2. A process as claimed in claim 1, wherein the isomerization is performed under neutral conditions.

3. A process as claimed in claim 1 or 2, wherein the unsaturated diol (II) is an unsaturated diol of the general formula (II) where the radicals R¹, R², R⁵ and R⁶ are hydrogen.

4. A process as claimed in claim 3, wherein the unsaturated diol (II) used is but-2-ene-1,4-diol.

5. A process as claimed in claims 1 to 4, wherein the rhenium catalyst used is rhenium heptoxide.

6. A process as claimed in claims 1 to 5, wherein the isomerization is carried out in at least one solvent selected from the group consisting of the C₅- to C₂₀-hydrocarbons, the halogenated C₁- to C₆-hydrocarbons and the C₂- to C₁₂-alkyl or aryl ethers.

7. A process as claimed in claims 1 to 6, wherein the vinyl glycol (I) formed is continuously removed from the reaction mixture by distillation.

## Revendications

1. Procédé de préparation de vinylglycols de la formule générale (I) 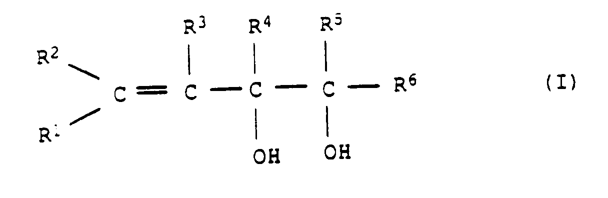 dans laquelle les symboles R¹ à R⁶ sont indépendamment l'un de l'autre identiques ou différents, ou peuvent être mutuellement attachés et représentent chacun un atome d'hydrogène, un radical alkyle en C₁ à C₁₀, cycloalkyle en C₅ à C₇, aralkyle en C₇ à C₁₀, ou un radical aryle à un ou deux noyaux, où ces restes ou radicaux peuvent aussi porter des substituants inertes, par l'isomérisation des diols correspondants de la formule générale (II) 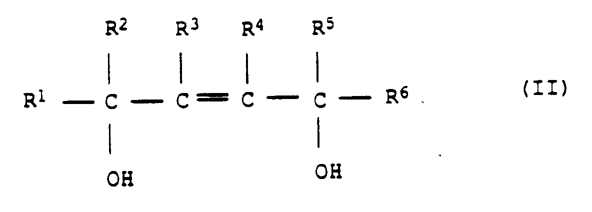 en présence de substances à activité catalytique, caractérisé en ce que l'on utilise, à titre de catalyseur, du rhénium ou un composé du rhénium.

2. Procédé suivant la revendication 1, caractérisé en ce que l'on entreprend l'isomérisation dans des conditions neutres.

3. Procédé suivant la revendication 1 ou 2, caractérisé en ce que l'on utilise, à titre de diol insaturé (II), un diol insaturé de la formule générale (II) dans laquelle les symboles R¹, R², R⁵ et R⁶ représentent chacun un atome d'hydrogène.

4. Procédé suivant la revendication 3, caractérisé en ce que l'on utilise le but-2-ène-1,4-diol à titre de diol insaturé (II).

5. Procédé suivant l'une quelconque des revendications 1 à 4, caractérisé en ce que l'on utilise de l'heptoxyde de rhénium à titre de catalyseur à base de rhénium.

6. Procédé suivant l'une quelconque des revendications 1 à 5, caractérisé en ce que l'on entreprend l'isomérisation dans au moins un solvant choisi dans le groupe formé par les hydrocarbures en C₅ à C₂₀, les hydrocarbures en C₁ à C₆ halogénés et les éthers aryliques ou alkyliques en C₂ à C₁₂.

7. Procédé suivant l'une quelconque des revendications 1 à 6, caractérisé en ce que l'on élimine en continu du mélange réactionnel, le vinylglycol (I) formé par distillation.
